(19) Europäisches Patentamt
European Patent Office
Office européen des brevets

(11) **EP 3 598 161 A1**

(12) **EUROPÄISCHE PATENTANMELDUNG**

(43) Veröffentlichungstag:
**22.01.2020 Patentblatt 2020/04**

(51) Int Cl.:
*G01R 33/54* *(2006.01)*   *G06N 3/04* *(2006.01)*

(21) Anmeldenummer: **18184274.1**

(22) Anmeldetag: **18.07.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder: **Zenge, Michael**
**90419 Nürnberg (DE)**

(54) **ERZEUGEN VON EINSTELLUNGSPARAMETERN FÜR EINE MAGNETRESONANZTOMOGRAPHIE-SEQUENZ MITTELS EINES BEDINGBAR ERZEUGENDEN KÜNSTLICHEN NEURONALEN NETZWERKS**

(57)     Die Erfindung stellt ein System und ein Verfahren zum Erzeugen von Einstellungsparametern (70) für eine durchzuführende MRT-Sequenz bereit, wobei ein bedingbares erzeugendes künstliches neuronales Netzwerk (22) verwendet wird, welches als Teil einer bedingbaren erzeugenden antagonistischen Netzwerkstruktur trainiert wurde. Die Erfindung stellt außerdem ein Verfahren zum Herstellen eines bedingbaren erzeugenden künstlichen neuronalen Netzwerks (22) bereit.

Das Bedingen des bedingbaren erzeugenden künstlichen neuronalen Netzwerks (22) kann zum Spezifizieren einer konkreten medizinischen Anwendung oder Fragestellung verwendet werden, um auf diese Anwendung oder Fragestellung hin optimierte Einstellungsparameter für eine MRT-Vorrichtung zu erhalten.

FIG 2

**Beschreibung**

Medizinische Bildgebung spielt eine wichtige Rolle bei der Diagnose und Behandlung von Krankheiten. Zu den am besten bekannten und etablierten medizinischen Bildgebungsverfahren gehört die Magnetresonanztomographie (MRT, engl. MRI von "magnetic resonance imaging").

Die Magnetresonanztomographie wird typischerweise in der Radiologie verwendet, um Bilder der Anatomie und/oder von physiologischen Vorgängen von sowohl gesunden als auch kranken Körpern zu bilden. MRT-Vorrichtungen (auch "MRT-Scanner" oder einfach "Scanner" genannt) verwenden starke magnetische Felder, z.B. stärker als 1 Tesla (1T), elektrische Feldgradienten und Hochfrequenzwellen, um Bilder von Körperteilen zu erzeugen.

Magnetresonanztomographiebilder werden üblicherweise in einer oder mehreren MRT-Sequenzen erzeugt. Eine MRT-Sequenz besteht aus mindestens einem, normalerweise mehreren, Hochfrequenzpulsen (engl. "RF pulses") gemäß Einstellungsparametern der MRT-Vorrichtung.

Einfach ausgedrückt, wird bei einer MRT-Prozedur ein Körper in ein statisches Magnetfeld eingebracht, das durch einen Magneten der MRT-Vorrichtung erzeugt wird. Für gewöhnlich wird der Körper dazu in einen Tunnel der MRT-Vorrichtung eingeführt. Als Reaktion auf das starke statische Magnetfeld richten sich die Spins der Wasserstoffkerne in dem Körper parallel zu dem Magnetfeld aus, wobei optional zusätzliche Vorbereitungen zur Präparierung der Magnetisierung der Wasserstoffkerne durchgeführt werden können.

Danach wird ein Hochfrequenzpuls von der MRT-Vorrichtung ausgesandt. Wenn dieser Hochfrequenzpuls mit der Larmorfrequenz ausgesandt wird, befindet er sich in Resonanz: es wird eine Phasenkohärenz in der Präzession der Wasserstoffkernspins erzeugt. Die Zeitdauer des Hochfrequenzpulses wird derart gewählt, dass die Spinmagnetisierung der Wasserstoffkerne in dem Körper senkrecht zu dem angelegten Magnetfeld angeordnet wird. In einer Empfangsspule der MRT-Vorrichtung, welche in der Nähe des Körpers angeordnet ist, wird durch die weiterhin rotierenden Wasserstoffkernspins (Larmor-Präzession) ein elektrischer Strom induziert, welcher auch als Magnetresonanzsignal (MR-Signal) bezeichnet wird.

Das MR-Signal schwindet mit der Zeit aufgrund von zwei Entspannungsprozessen. Der Kohärenzverlust des Spinsystems schwächt das MR-Signal mit einer Zeitkonstante ab, welche üblicherweise "transversale Relaxationszeit" genannt und mit "T2" bezeichnet wird. Gleichzeitig entspannt sich der Magnetisierungsvektor allmählich hin zu seiner Gleichgewichtsausrichtung parallel zu dem Magnetfeld. Dies geschieht mit einer Zeitkonstante, welche "Spin-Gitter-Relaxationszeit" oder "longitudinale Relaxationszeit" genannt und mit "T1" bezeichnet wird.

Eine räumliche Auflösung des MR-Signals wird durch die Verwendung eines magnetischen Feldgradienten (d.h. eines Gradienten des magnetischen Felds) erzeugt, welcher bewirkt, dass die Wasserstoffkernspins an verschiedenen Orten (üblicherweise in verschiedenen Schichten des Körpers) mit leicht unterschiedlicher Geschwindigkeit präzessieren. Dies kann verwendet werden, um Signalursprünge innerhalb des Körpers zu lokalisieren bzw. aufzulösen.

Der Kontrast in MRT-Bildern entsteht dadurch, dass unterschiedliche Bestandteile des Körpers (insbesondere Knochen, Gewebe...) im Allgemeinen unterschiedliche Relaxationszeiten T1 und T2 aufweisen. Da dies besonders für weiche Gewebetypen gilt, weisen MRT-Bilder üblicherweise eine gute Auflösung für solche weichen Gewebe auf. Durch das Auswählen optimaler MRT-Sequenzen (oder, mit anderen Worten, durch Durchführen des MRT-Prozesses mit optimal gewählten Einstellungsparametern) kann somit ein Kontrast für ein interessierendes Gewebe oder einen interessierenden Körperteil (z.B. ein bestimmtes Organ) maximiert werden. Hierdurch können sich die Chancen für eine erfolgreiche Diagnose und Behandlung verbessern.

Generell werden mehrere MRT-Sequenzen benötigt, um ein Gewebe angemessen abzubilden. Eine Kombination von MRT-Sequenzen (häufig zusammen mit bestimmten zu untersuchenden Orten und/oder Ausrichtungen bzw. Schnittebenen) wird häufig als MRT-Protokoll bezeichnet. Ein Benutzer der MRT-Vorrichtung (häufig ein Radiologe) passt die MRT-Sequenzen an, um die bestmögliche Bildgebung für eine bestimmte medizinische Fragestellung bereitzustellen.

Eine MRT-Sequenz wird üblicherweise durch mehrere Einstellungsparameter beschrieben, umfassend beispielsweise, zum Teil je nach konkret durchgeführter MRT-Sequenz:

- eine Echozeit (TE)
- eine Repetitionszeit (TR)
- einen Anregungswinkel (engl. flip angle)
- eine Bandbreite des Hochfrequenzpulses
- einen Turbofaktor (auch Puls-Zug-Länge genannt)
- einen Gradienten bei der Hochfrequenzpuls-Anregung (engl. "spoiling gradient" oder "Crusher gradient")
- und/oder dergleichen mehr.

Zwei entscheidende Einstellungsparameter sind die Repetitionszeit (üblicherweise mit TR abgekürzt) und die Echozeit (üblicherweise mit TE abgekürzt), welche normalerweise in Millisekunden gemessen und angegeben werden (so auch beispielsweise in den nachfolgend abgedruckten Codeabschnitten).

Die Echozeit, TE, gibt die Zeitdauer vom Zentrum des Hochfrequenzpulses bis zum Zentrum des Echos, d.h. des induzierten Signals, an. Für MRT-Sequenzen mit mehreren Echos zwischen jedem Hochfrequenzpuls können mehrere Echozeiten definiert werden, welche üblicherweise als TE1, TE2, TE3 etc. bezeichnet werden.

Die Repetitionszeit, TR, ist die Zeitdauer zwischen einander entsprechenden aufeinanderfolgenden Punkten einer wiederkehrenden Serie von Hochfrequenzpulsen (bzw. Echos).

Zu den am häufigsten verwendeten MRT-Sequenzen gehören die sogenannte T1-gewichtete MRT-Sequenz (oder: "T1-Gewichtung"), die sogenannte T2-gewichtete MRT-Sequenz (oder: "T2-Gewichtung"), und die Protonendichte-gewichtete MRT-Sequenz.

Bei der T1-Gewichtung ist üblicherweise die Repetitionszeit, TR, kurz im Verhältnis zu der längsten longitudinalen Relaxationszeit, T1, eines interessierenden Gewebes, und die Echozeit, TE, ist kurz im Verhältnis zu der transversalen Relaxationszeit, T2, des interessierenden Gewebes. Auf diese Weise werden T2-Beiträge zu dem Bildkontrast verringert.

Bei der T2-Gewichtung ist üblicherweise die Repetitionszeit, TR, lang im Vergleich zur longitudinalen Relaxationszeit, T1, des Gewebes (um T1-Beiträge zu verringern), und die Echozeit, TE, wird zwischen der längsten und der kürzesten transversalen Relaxationszeit, T2, der interessierenden Gewebe gewählt.

Es versteht sich, dass eine bestimmte MRT-Sequenz für eine bestimmte Art von Gewebe Tl-gewichtet sein kann und für eine andere Art von Gewebe eine andere Art von Gewichtung aufweist.

Eine Protonendichte-gewichtete MRT-Sequenz kann durch Verwenden einer langen Repetitionszeit, TR, und einer kurzen Echozeit, TE, erzielt werden.

Für eine detailliertere Diskussion der technischen Hintergründe für MRT-Verfahren wird auf R.W.Brown et al.: "Magnetic Resonance Imaging: Physical Principles and Sequence Design", Wiley-Blackwell, 2nd edition (June 23, 2014), ISBN-13: 978-0471720850, verwiesen.

Für viele Krankheiten und/oder Körperteile gibt es spezifische MRT-Protokolle. Das Erstellen von optimalen MRT-Sequenzen und MRT-Protokollen erfordert bislang viele Jahre der Übung und Erfahrung, insbesondere wenn ein Kontrast optimal auf eine konkrete medizinische Fragestellung (z.B. Anwesenheit eines Tumors) abgestimmt sein soll.

Da ausreichend ausgebildetes Personal nicht stets zur Stelle ist, wird eine Reduktion der Komplexität der Bedienung von MRT-Vorrichtungen und der Benutzerschnittstelle einer MRT-Vorrichtung angestrebt.

Aus dem Gebiet des Maschinenlernens und der künstlichen Intelligenz sind erzeugende (d.h. generierende) antagonistische Netzwerkstrukturen (engl. "generative adversarial networks", GAN) bekannt, insbesondere aus der wissenschaftlichen Veröffentlichung von I. J. Goodfellow et al.: "Generative Adversarial Nets", in: Advances in Neural Information Processing Systems 27 (NIPS 2014), edited by Z. Ghahramani et al., erhältlich unter der URL: https://papers.nips.cc/paper/5423-generative-adversarial-nets, welche im Folgenden als "Goodfellow et al." zitiert werden wird.

In Goodfellow et al., ist eine erzeugende antagonistische Netzwerkstruktur beschrieben, in welcher zwei Netzwerke im Wesentlichen gleichzeitig, als Antagonisten, trainiert werden: ein erzeugender (engl. "generative") Teil, d.h. ein erzeugendes künstliches neuronales Netzwerk, und ein unterscheidender (engl. "discriminative") Teil, d.h. ein unterscheidendes künstliches neuronales Netzwerk.

Das erzeugende künstliche neuronale Netzwerk wird trainiert, basierend auf einer ursprünglichen Datenverteilung (Trainingsdaten) eine eigene Datenverteilung zu erzeugen. Das unterscheidende künstliche neuronale Netzwerk wird trainiert, zu erkennen, ob ein bestimmter Datensatz zu der ursprünglichen Datenverteilung gehört oder zu der von dem erzeugenden künstlichen neuronalen Netzwerk erzeugten Datenverteilung. Eine Idealsituation ist erreicht, wenn das unterscheidende künstliche neuronale Netzwerk nicht mehr in der Lage ist, die Datensätze der ursprünglichen Datenverteilung von denen der erzeugten Datenverteilung zu unterscheiden.

Ein spezielles Beispiel von erzeugenden antagonistischen Netzwerkstrukturen sind bedingbare erzeugende antagonistische Netzwerkstrukturen (engl. "conditional generative adversarial nets"), welche insbesondere in der wissenschaftlichen Veröffentlichung von M. Mirza et al.: "Conditional Generative Adversarial Nets", arXiv preprint arXiv:1411.1784v1, submitted on 6 November 2014, erhältlich unter der URL: https://arxiv.org/abs/1411.1784, beschrieben sind. Diese Veröffentlichung wird im Folgenden als "Mirza et al." zitiert.

In bedingbaren erzeugenden antagonistischen Netzwerkstrukturen wird ein bedingendes Element (normalerweise ein Vektor, d.h. ein "Bedingungsvektor") sowohl bei dem erzeugenden künstlichen neuronalen Netzwerk als auch bei dem unterscheidenden künstlichen neuronalen Netzwerk eingeführt, wodurch Datensätze erzeugt (bzw. unterschieden) werden können, welche auf verschiedene Situationen oder Zielbereiche (welche durch die Bedingungen modelliert werden) hin angepasst sind. Das Setzen einer solchen Bedingung wird vorliegend hauptsächlich als "Bedingen" bezeichnet, und die "Bedingbarkeit" des Netzwerks bedeutet, dass es in der Lage ist, solche Bedingungen zu erhalten und seine Ausgabe unter der/den gegebenenfalls gesetzten Bedingung/en zu erzeugen.

Eine weitere bekannte Variante von erzeugenden antagonistischen Netzwerkstrukturen sind so genannte erzeugende antagonistische Netzwerkstrukturen basierend auf der Methode der kleinsten Quadrate (engl. "least squares generative adversarial network"), welche beispielsweise in der wissenschaftlichen Veröffentlichung von X. Mao et al.:

"Least Squares Generative Adversarial Networks", arXiv preprint arXiv:1611.04076v3, submitted on 13 November 2016, last revised on 5 April 2017, erhältlich unter der URL: https://arxiv.org/abs/1611.04076 beschrieben sind. Diese Veröffentlichung wird im Folgenden als "Mao et al." zitiert werden.

Es ist eine Aufgabe der vorliegenden Erfindung, verbesserte Systeme und Verfahren zum Erzeugen von Einstellungsparametern für eine von einer MRT-Vorrichtung durchzuführende MRT-Sequenz bereitzustellen.

Diese Aufgabe wird gelöst durch die Aspekte der vorliegenden Erfindung.

Dementsprechend stellt die Erfindung gemäß einem ersten Aspekt ein System zum Erzeugen von Einstellungsparametern für eine durchzuführende Magnetresonanztomographie-(MRT-)Sequenz bereit, umfassend:

eine Eingabeschnittstelle, welche dazu eingerichtet ist, Eingabedaten bezüglich der durchzuführenden MRT-Sequenz zu erhalten;

einer Recheneinrichtung, welche dazu ausgebildet ist, ein bedingbares erzeugendes künstliches neuronales Netzwerk, zu implementieren,

wobei das bedingbare erzeugende künstliche neuronale Netzwerk dazu ausgebildet und trainiert ist, basierend auf den von der Eingabeschnittstellen empfangenen Eingabedaten Einstellungsparameter für die durchzuführende MRT-Sequenz zu erzeugen;

wobei das bedingbare erzeugende künstliche neuronale Netzwerk bedingbar ist, um Einstellungsparameter für zumindest jeweils eine T1-gewichtete MRT-Sequenz, eine T2-gewichtete MRT-Sequenz und eine Protondichte-(PD-)gewichtete MRT-Sequenz zu erzeugen.

Das System kann in eine MRT-Vorrichtung integriert sein. Dementsprechend stellt die Erfindung gemäß einem weiteren Aspekt auch eine MRT-Vorrichtung bereit, welche das System gemäß dem ersten Aspekt sowie weitere Elemente umfasst, beispielsweise einen Magnetfeldgenerator (Elektromagneten), einen Hochfrequenzpulsgenerator, einen Tunnel, eine Steuereinrichtung und dergleichen mehr.

Bei der Recheneinrichtung kann es sich um jegliche Vorrichtung, oder jegliches Mittel, zum Rechnen handeln, d.h. zum Ausführen einer Software, einer App, oder eines Algorithmus'. Beispielsweise kann es sich bei der Recheneinrichtung um einen einzelnen Prozessorkern, einen Mikroprozessor, eine anwendungsspezifische integrierte Schaltung (ASIC) oder dergleichen handeln. Bevorzugt umfasst die Recheneinrichtung eine Anordnung von Prozessorkernen, z.B. von zentralen Prozessorkernen (CPUs) und/oder von graphischen Prozessorkernen (GPUs)

Die Recheneinrichtung kann auch teilweise oder vollständig durch miteinander verbundene, entfernt angeordnete Geräte realisiert sein, wie etwa durch Cloud-Rechenserver und/oder al seine virtuelle Maschine. Die Recheneinrichtung kann auch in eine Steuereinrichtung einer MRT-Vorrichtung integriert sein.

Das bedingbare erzeugende künstliche neuronale Netzwerk ist bevorzugt als ein feed-forward-Netzwerk ausgebildet, besonders bevorzugt als ein Multischicht-Perzeptron. Dies ermöglicht ein besonders einfaches Trainieren des neuronalen Netzwerks mittels backpropagation.

Das bedingbare erzeugende künstliche neuronale Netzwerk kann insbesondere dadurch bedingt werden (d.h. mit Bedingungen versehen werden, die zu erfüllen sind), dass dessen ursprüngliche Eingabevektoren mit einem entsprechenden Bedingungsvektor zusammengekettet (engl. "concatenated") werden, wobei der Bedingungsvektor eine Art von MRT-Sequenz indiziert (z.B. T1-gewichtete MRT-Sequenz, T2-gewichtete MRT-Sequenz, PD-gewichtete MRT-Sequenz), zu welcher der jeweilige ursprüngliche Eingabevektor gehört. Wie im Zusammenhang mit Mirza et al. bereits erwähnt wurde, wird derselbe Typ von Bedingungsvektor sowohl für das erzeugende, als auch für das unterscheidende bedingte neuronale Netzwerk verwendet. Dies wird im Folgenden insbesondere im Zusammenhang mit Codebeispielen noch veranschaulicht werden.

Der Bedingungsvektor c kann beispielsweise derart aufgebaut sein, dass er einen Eintrag mit einem Wert von "1" an einer Stelle $c_i$ aufweist, welche eine erste Art von MRT-Sequenz indiziert (z.B. T1-gewichtete MRT-Sequenz, T2-gewichtete MRT-Sequenz, PD-gewichtete MRT-Sequenz), und dass er an allen anderen Stellen $c_{j\neq i}$ Einträge mit einem Wert von "0" aufweist. Beispielsweise kann ein Vektor mit der Gestalt $(1,0,0)^T$ eine T1-gewichtete MRT-Sequenz, ein Vektor mit der Gestalt $(0,1,0)^T$ eine T2-gewichtete MRT-Sequenz, und ein Vektor mit der Gestalt $(0,0,1)^T$ eine PD-gewichtete MRT-Sequenz indizieren.

Der Bedingungsvektor kann auch eine oder mehrere weitere Bedingungen enthalten, beispielsweise eine Bedingung, welche ein zu untersuchendes Körperteil, einen Körperfettanteil, eine gewünschte Artefaktunterdrückung oder dergleichen indiziert.

Der Erfinder hat herausgefunden, dass sich ein bedingbares erzeugendes künstliches neuronales Netzwerk gut für das spezielle technische Gebiet des Erzeugens von Einstellungsparametern für MRT-Sequenzen eignet, und mit einer Vielzahl von Varianten und Optionen optimal auf diese Aufgabe hin angepasst werden kann. Die Erfindung ermöglicht somit das Erzeugen von optimalen Einstellungsparametern für MRT-Sequenzen basierend auf Trainingsdaten, ohne dass die Beteiligung eines Radiologen notwendig ist.

Die Erfindung stellt außerdem, gemäß einem zweiten Aspekt, ein Verfahren zum Herstellen eines Systems zum Erzeugen von Einstellungsparameter für eine durchzuführende MRT-Sequenz bereit, insbesondere zum Herstellen eines Systems gemäß dem ersten Aspekt der Erfindung. Das Verfahren gemäß dem zweiten Aspekt umfasst dabei zumindest die Schritte:

Bereitstellen von Trainingsdaten, welche Eingabedaten bezüglich der durchzuführenden MRT-Sequenz umfassen und welche entsprechende Einstellungsparameter als Labels umfassen;

Bereitstellen eines bedingbaren erzeugenden künstlichen neuronalen Netzwerks und eines bedingbaren unterscheidenden künstlichen neuronalen Netzwerks;

Trainieren des bedingbaren erzeugenden künstlichen neuronalen Netzwerks unter Verwendung der bereitgestellten Trainingsdaten, wobei das bedingbare erzeugende künstliche neuronale Netzwerk trainiert wird, die Einstellungsparameter für durchzuführende MRT-Sequenzen basierend auf den Eingabedaten der Trainingsdaten zu erzeugen; wobei das bedingbare erzeugende künstliche neuronale Netzwerk bedingbar ist, um Einstellungsparameter für zumindest jeweils eine T1-gewichtete MRT-Sequenz, eine T2-gewichtete MRT-Sequenz und eine Protondichtegewichtete MRT-Sequenz zu erzeugen;

Trainieren des bedingbaren unterscheidenden künstlichen neuronalen Netzwerk unter Verwendung der bereitgestellten Trainingsdaten und der von dem bedingbaren erzeugenden künstlichen neuronalen Netzwerk erzeugten Einstellungsparameter, wobei das bedingbare unterscheidende künstliche neuronale Netzwerk trainiert wird, um für jeden einzelnen Parametersatz zu unterscheiden, ob dieser zu den Trainingsdaten oder zu den von dem bedingbaren erzeugenden künstlichen neuronalen Netzwerk erzeugten Einstellungsparametern gehört;

und wobei das Trainieren des bedingbaren erzeugenden künstlichen neuronalen Netzwerks umfasst, das bedingbare erzeugende künstliche neuronale Netzwerk derart zu trainieren, die Einstellungsparameter derart zu erzeugen, dass das bedingbare unterscheidende künstliche neuronale Netzwerk die Trainingsdaten nicht von den von dem bedingbaren erzeugenden künstlichen neuronalen Netzwerk erzeugten Einstellungsparametern unterscheiden kann;

Bereitstellen und Einrichten einer Eingabeschnittstelle zum Empfangen von Eingabedaten bezüglich durchzuführender MRT-Sequenzen; und

Bereitstellen und Einrichten einer Recheneinrichtung zum Implementieren des trainierten bedingbaren erzeugenden künstlichen neuronalen Netzwerks zum Erzeugen der Einstellungsparameter für die durchzuführenden MRT-Sequenz basierend auf den empfangenen Eingabedaten.

Das Verfahren gemäß dem zweiten Aspekt eignet sich insbesondere zur Herstellung eines Systems gemäß dem ersten Aspekt. Das Verfahren gemäß dem zweiten Aspekt ist vorteilhaft gemäß allen mit Bezug auf das System gemäß dem ersten Aspekt beschriebenen Varianten, Modifikationen, Ausführungsformen und Weiterbildungen anpassbar und umgekehrt.

Die Erfindung stellt weiterhin, gemäß einem dritten Aspekt, ein Verfahren zum Erzeugen von Einstellungsparametern für eine durchzuführende MRT-Sequenz bereit, umfassend zumindest die Schritte:

Empfangen von Eingabedaten bezüglich der durchzuführenden MRT-Sequenz;

Erzeugen, unter Verwendung eines trainierten bedingbaren erzeugenden künstlichen neuronalen Netzwerks, von Einstellungsparametern für die durchzuführende MRT-Sequenz basierend auf den empfangenen Eingabedaten; wobei das bedingbare erzeugende künstliche neuronale Netzwerk bedingbar ist, um Einstellungsparameter für zumindest jeweils eine T1-gewichtete MRT-Sequenz, eine T2-gewichtete MRT-Sequenz und eine Protondichtegewichtete MRT-Sequenz zu erzeugen.

Das Verfahren gemäß dem dritten Aspekt eignet sich insbesondere zur Durchführung mit dem System gemäß dem ersten Aspekt. Das Verfahren gemäß dem dritten Aspekt ist vorteilhaft gemäß allen mit Bezug auf das System gemäß dem ersten Aspekt beschriebenen Varianten, Modifikationen, Ausführungsformen und Weiterbildungen anpassbar und umgekehrt.

Die Erfindung stellt weiterhin, gemäß einem vierten Aspekt, ein Computerprogrammprodukt bereit, welches ausführbaren Programmcode umfasst, welcher dazu ausgebildet ist, das Verfahren gemäß einer Ausführungsform des dritten Aspekts der vorliegenden Erfindung durchzuführen, wenn er ausgeführt wird.

Außerdem stellt die Erfindung, gemäß einem fünften Aspekt, ein nicht-flüchtiges Datenspeichermedium bereit, welches ausführbaren Programmcode umfasst, welcher dazu ausgebildet ist, das Verfahren gemäß einer Ausführungsform des dritten Aspekts der vorliegenden Erfindung durchzuführen, wenn er ausgeführt wird. Bei dem Datenspeichermedium kann es sich insbesondere um eine Festplatte, einen Memorystick, eine DVD, eine BluRay-Disc und dergleichen handeln.

Außerdem stellt die Erfindung, gemäß einem sechsten Aspekt, einen Datenstrom bereit, welcher ausführbaren Programmcode umfasst oder repräsentiert, welcher dazu ausgebildet ist, das Verfahren gemäß einer Ausführungsform des dritten Aspekts der vorliegenden Erfindung durchzuführen, wenn er ausgeführt wird.

Weitere vorteilhafte Ausführungsformen, Varianten und Weiterbildungen ergeben sich aus den Unteransprüchen sowie aus der Beschreibung im Zusammenhang mit den Figuren.

In einigen vorteilhaften Ausführungsformen umfassen die Eingabedaten zumindest einen der folgenden Prozentsätze:

- einen Prozentsatz, zu welchem die durchzuführende MRT-Sequenz T1-gewichtet sein soll
- einen Prozentsatz, zu welchem die durchzuführende MRT-Sequenz T2-gewichtet sein soll
- einen Prozentsatz, zu welchem die durchzuführende MRT-Sequenz Protondichte-gewichtet sein soll.

Werte für diese Eingabedaten (oder: "Eingabeparameter") können beispielsweise in Tabellen für verschiedene medizinische Aufgabenstellungen gespeichert werden und können dementsprechend auch von ungeschultem Personal in das System eingegeben werden. Das System kann diese Eingabeparameter empfangen und darunter, optional unter Verwendung weiterer Eingabeparameter, entsprechende Einstellungsparameter für eine MRT-Vorrichtung generieren.

Besonders vorteilhaft werden genau zwei der drei genannten Prozentsätze als Eingabedaten verwendet, wobei sich der entsprechende dritte Prozentsatz aus der Differenz zwischen Einhundert und der Summe der beiden verwendeten Prozentsätze ergibt. Mit anderen Worten soll die Summe der drei genannten Prozentsätze Einhundert ergeben. In dieser Weise können mit einfachen Mitteln große Bereiche der in medizinischen Anwendungen verwendeten MRT-Sequenzen abgedeckt werden.

In Fällen, in denen das System dazu ausgelegt ist, Einstellungsparameter für mehr als nur TI-gewichtete, T2-gewichtete und PD-gewichtete MRT-Sequenzen zu erzeugen, können entsprechend weitere Prozentsätze für die entsprechenden Gewichtungen als Eingabedaten verwendet werden, wobei wieder vorgesehen sein kann, dass sich die Summe aller einstellbaren Prozentsätze zu Einhundert addiert.

In einigen vorteilhaften Ausführungsformen umfassen die Einstellungsparameter eine Repetitionszeit, TR, und/oder eine Echozeit, TE, der durchzuführenden MRT-Sequenz. Mit anderen Worten ist bei diesen Ausführungsformen das System dazu eingerichtet, zumindest auch eine Repetitionszeit und/oder eine Echozeit der MRT-Sequenz zu erzeugen.

In einigen bevorzugten Ausführungsformen bestehen die Einstellungsparameter aus der Repetitionszeit, TR, und der Echozeit, TE. In diesem Fall kann eine Ausgabeschicht (engl. "output layer") des bedingbaren erzeugenden künstlichen neuronalen Netzwerks aus zwei Ausgabeknoten bestehen, von denen der erste einen Wert für die Repetitionszeit, TR, ausgibt und der zweite einen Wert für die Echozeit, TE ausgibt. Der Erfinder hat herausgefunden, dass bereits durch Erzeugen dieser beiden Einstellungsparameter mit optimalen Werten ein zufriedenstellendes Gesamtergebnis der MRT-Sequenz erzielt wird.

Wenn die durch das System erzeugten Einstellungsparameter erweitert werden, um mehr Einstellungsparameter als nur die Repetitionszeit, TR, und die Echozeit, TE, zu erzeugen, kann dies den benötigten Rechenaufwand (und damit möglicherweise auch den benötigten Hardwareaufwand) erhöhen. Andererseits wird dies die Qualität des Ergebnisses der MRT-Sequenzen weiter verbessern und wird insbesondere bei besonders schwierigen (z.B. von Haus aus kontrastarmen) Bildgebungsaufgaben vorteilhaft sein.

In einigen vorteilhaften Ausführungsformen umfassen die Einstellungsparameter einen Turbofaktor und/oder eine Bandbreite der durchzuführenden MRT-Sequenz. Der Turbofaktor (auch "Pulszuglänge" genannt) bezeichnet die Anzahl von Echos, welche in einer Reihe von Echo-Bildgebungsverfahren zu einem einzigen Bild oder einer Gruppe von Bildern kombiniert werden. Solche Verfahren umfassen beispielsweise "rapid acquisition with relaxation enhancement" (RARE), "fast-spin echo" (FSE) sowie "turbo spin-echo" (TSE). Der Erfinder hat herausgefunden, dass sich auch diese Einstellungsparameter besonders zum Erzeugen durch das erfindungsgemäße System eignen.

In einigen vorteilhaften Ausführungsformen kann das bedingbare erzeugende künstliche neuronale Netzwerk durch eine magnetische Feldstärke der durchzuführenden MRT-Sequenz bedingt sein, d.h., das bedingbare erzeugende künstliche neuronale Netzwerk kann dazu trainiert und eingerichtet sein, die Einstellungsparameter (falls die entsprechende Bedingung gesetzt wird) zu erzeugen unter der Bedingung, dass ein bestimmte magnetische Feldstärke durch die MRT-Vorrichtung erzeugt wird (insbesondere für die Feldstärkenwerte 1.5 Tesla und 3 Tesla). Die optimalen Einstellungsparameter verändern sich nämlich in Abhängigkeit von der magnetischen Feldstärke, welche durch die MRT-Vorrichtung angelegt wird. Das erfindungsgemäße System eignet sich gut dafür, diese Änderungen zu berechnen.

In dem Fall, dass das System eine MRT-Vorrichtung umfasst, oder in eine MRT-Vorrichtung integriert ist, kann somit vorteilhaft diese MRT-Vorrichtung so ausgebildet sein, dass, wenn ein Benutzer eine Einstellung für die magnetische Feldstärke ändert, das System automatisch unter Verwendung des bedingbaren erzeugenden künstlichen neuronalen Netzwerks entsprechend angepasste neue optimale Einstellungsparameter erzeugt.

In einigen vorteilhaften Ausführungsformen ist das bedingbare erzeugende künstliche neuronale Netzwerk zusätzlich bedingbar durch mindestens eine der folgenden Bedingungen:

- eine Information über einen Ort und/oder eine Ausrichtung einer Schnittebene durch einen Körper, an welchem die MRT-Sequenz durchzuführen ist,

- eine Information über einen Ort und/oder einen Körperteil eines Körper, an welchem die MRT-Sequenz durchzuführen ist,
- einen Körperfettanteil,
- eine gewünschte Artefaktunterdrückung.

Typische Schnittebenenausrichtungen sind die transversale Ausrichtung, die sagittale Ausrichtung sowie die koronare Ausrichtung. Hierdurch können die erzeugten Einstellungsparameter noch besser auf die medizinische Aufgabenstellung (z.B. Diagnose) angepasst werden.

Körperteile können beispielsweise ein menschliches Knie, ein menschliches Gehirn, ein menschliches Herz und dergleichen umfassen. Auch hierdurch können die erzeugten Einstellungsparameter noch besser auf die medizinische Aufgabenstellung angepasst werden.

In einigen vorteilhaften Ausführungsformen wurde das bedingbare erzeugende künstliche neuronale Netzwerk als Teil einer bedingbaren erzeugenden antagonistischen Netzwerkstruktur trainiert, wobei die bedingbare erzeugende antagonistische Netzwerkstruktur das bedingbare erzeugende künstliche neuronale Netzwerk als ihren erzeugenden Teil umfasst und ein bedingbares unterscheidendes künstliches neuronales Netzwerk als ihren unterscheidenden Teil umfasst.

Zu den Grundlagen von antagonistischen Netzwerkstrukturen wird beispielhaft nochmals auf Goodfellow et al. sowie auf Mirza et al. verwiesen, wobei das bedingbare erzeugende künstliche neuronale Netzwerk der vorliegenden Erfindung als "generative model" bzw. "generative network" fungiert und das bedingbare unterscheidende künstliche neuronale Netzwerk als "discriminative model" bzw. "discriminative network" fungiert.

Es hat sich überraschender Weise herausgestellt, dass diese Technik für die vorliegende spezifische technische Anwendung, nämlich das Erzeugen von Einstellungsparametern für MRT-Sequenzen, mit sehr guten Ergebnissen anwenden lässt.

In einigen vorteilhaften Ausführungsformen besteht das bedingbare erzeugende künstliche neuronale Netzwerk aus einer Eingabeschicht, einer Ausgabeschicht und einer einzelnen, dazwischenliegenden verborgenen Schicht (engl. "hidden layer"). Es hat sich herausgestellt, dass seine solche vergleichsweise einfache Anordnung bereits ausreichen kann, den komplexen Anforderungen an Einstellungsparameter für MRT-Sequenzen zu genügen. Vorteilhaft umfasst die verborgene Schicht mehr als fünf Knoten, bevorzugt mehr als zehn Knoten, besonders bevorzugt mehr als zwanzig Knoten, beispielsweise vierzig Knoten oder mehr, z.B. achtundvierzig Knoten.

Die Ausgabeschicht besteht vorteilhaft aus je einem Knoten pro zu erzeugendem Einstellungsparameter. Im Fall des Erzeugens bloß der Echozeit, TE, und der Repetitionszeit, TR, kann die Ausgabeschicht somit vorteilhaft aus genau zwei Knoten bestehen.

In einigen vorteilhaften Ausführungsformen umfasst das bedingbare erzeugende künstliche neuronale Netzwerk eine Eingabeschicht, eine Ausgabeschicht und mindestens eine verborgene Schicht (engl. "hidden layer" oder "latent layer"). Die Eingabeschicht und die mindestens eine verborgene Schicht können zumindest einen Teil eines Auto-Encoder-Netzwerk bilden. Solche Netzwerke bilden einen hochdimensionalen Eingabevektor in einem -üblicherweise- niedrigerdimensionalen verborgenen Raum (engl. "latent space") ab. Solche Auto-Encoder können somit dazu verwendet werden, den Rechenaufwand zu verringern, indem weniger relevante Daten (bzw. Einträge des Eingabevektors) eliminiert oder nur in geringem Maße in dem verborgenen Raum berücksichtigt werden. Auto-Encoder können auch dazu verwendet werden, fehlende Informationen in dem Eingabevektor zu intrapolieren.

In einigen vorteilhaften Ausführungsformen wird das Trainieren des bedingbaren erzeugenden künstlichen neuronalen Netzwerks und/oder das Trainieren des bedingbaren unterscheidenden künstlichen neuronalen Netzwerks unter Verwendung einer Verlustfunktion durchgeführt, welche auf der Methode der kleinsten Quadrate basiert.

Die obigen Ausgestaltungen und Weiterbildungen lassen sich, sofern sinnvoll, beliebig miteinander kombinieren. Weitere mögliche Ausführungsformen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

Die vorliegende Erfindung wird nachfolgend anhand der in den schematischen Figuren angegebenen Ausführungsbeispiele näher erläutert. Es zeigen dabei:

Fig. 1    ein schematisches Blockschaltbild eines Systems gemäß einer Ausführungsform des ersten Aspekts der vorliegenden Erfindung;

Fig. 2    eine schematische Darstellung des trainierten bedingbaren erzeugenden künstlichen neuronalen Netzwerks des Systems gemäß Fig. 1;

Fig. 3    ein schematisches Flussdiagramm zum Erläutern eines Verfahrens zum Herstellen eines System zum Erzeugen von Einstellungsparametern für eine durchzuführende MRT-Sequenz gemäß einer Ausführungsform des zweiten Aspekts der vorliegenden Erfindung;

Fig. 4    ein schematisches Flussdiagramm zum Erläutern eines Verfahrens zum Erzeugen von Einstellungsparametern für eine durchzuführende MRT-Sequenz gemäß einer Ausführungsform des dritten Aspekts der vorliegenden Erfindung;

Fig. 5    eine schematische Darstellung eines Computerprogrammprodukts gemäß einer Ausführungsform des vierten Aspekts der vorliegenden Erfindung; und

Fig. 6    eine schematische Darstellung eines nichtflüchtigen Datenspeichermediums gemäß einer Ausführungsform des fünften Aspekts der vorliegenden Erfindung.

Die beiliegenden Figuren sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts anderes ausgeführt ist - je-weils mit denselben Bezugszeichen versehen.

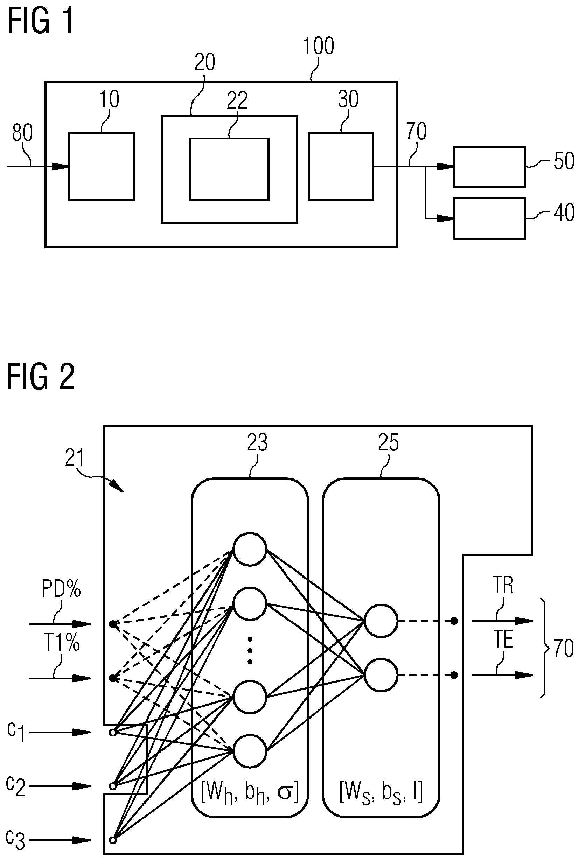

Fig. 1 zeigt ein schematisches Blockschaltbild eines Systems 100 gemäß einer Ausführungsform des ersten Aspekts der vorliegenden Erfindung zum Erzeugen von Einstellungsparametern 70 für eine durchzuführende Magnetresonanztomographie-(MRT-)Sequenz.

Das System 100 umfasst eine Eingabeschnittstelle 10, welche dazu eingerichtet ist, Eingabedaten 80 (oder: "Eingabedaten") bezüglich der durchzuführenden MRT-Sequenz zu erhalten.

Die Eingabedaten 80 umfassen in der vorliegenden Ausführungsform einen Prozentsatz PD%, zu welchem die durchzuführende MRT-Sequenz PD-gewichtet sein soll, sowie einen zweiten Prozentsatz T1%, zu welchem die durchzuführende MRT-Sequenz T1-gewichtet sein soll. Das System 100 ist weiterhin derart konfiguriert dass die durchzuführende MRT-Sequenz dann zu einem dritten Prozentsatz, T2%, T2-gewichtet sein soll, wobei gelten soll:

$$T1\% + T2\% + PD\% = 1$$

Dementsprechend genügen zwei der drei Prozentsätze T1%, T2% und PD%, nämlich PD% und T1%, als Eingabedaten 80, um die Gewichtung der durchzuführenden MRT-Sequenz in allen drei Prozentsätzen festzulegen. Eine Einstellung von PD%=100, T1%=0 beschreibt somit eine vollständig PD-gewichtete MRT-Sequenz und bedingt somit T2%=0, eine Einstellung von PD%=50, T1%=25 eine Einstellung von T2%=25 und so weiter.

Das System 100 umfasst weiterhin eine Recheneinrichtung 20, welche dazu ausgebildet ist, ein bedingbares erzeugendes künstliches neuronales Netzwerk 22 (engl. "conditional generative artificial neural network") zu implementieren.

Das bedingbare erzeugende künstliche neuronale Netzwerk 22 ist dazu ausgebildet und trainiert, basierend auf den von der Eingabeschnittstellen 10 empfangenen Eingabedaten 80 die Einstellungsparameter 71 für die durchzuführende MRT-Sequenz zu erzeugen. Das bedingbare erzeugende künstliche neuronale Netzwerk 22 ist derart bedingt, dass es dazu in der Lage ist Einstellungsparameter für zumindest jeweils eine T1-gewichtete MRT-Sequenz, eine T2-gewichtete MRT-Sequenz und eine Protondichte-(PD-)gewichtete MRT-Sequenz zu erzeugen.

Das bedingbare erzeugende künstliche neuronale Netzwerk 22 kann insbesondere dadurch bedingt werden (d.h. mit Bedingungen versehen werden, die zu erfüllen sind), dass dessen ursprüngliche Eingabevektoren (vorliegend also beispielsweise ein Vektor mit einem ersten Eintrag, welcher PD% indiziert oder angibt und mit einem zweiten Eintrag, welcher T1% indiziert oder angibt) mit einem entsprechenden Bedingungsvektor zusammengekettet (engl. "concatenated") werden, wobei der Bedingungsvektor eine Art von MRT-Sequenz indiziert (z.B. T1-gewichtete MRT-Sequenz, T2-gewichtete MRT-Sequenz, PD-gewichtete MRT-Sequenz), zu welcher der jeweilige ursprüngliche Eingabevektor gehört.

Der Bedingungsvektor c kann beispielsweise derart aufgebaut sein, dass er einen Eintrag mit einem Wert von "1" an einer Stelle $c_i$ aufweist, welche eine erste Art von MRT-Sequenz indiziert (z.B. T1-gewichtete MRT-Sequenz, T2-gewichtete MRT-Sequenz, PD-gewichtete MRT-Sequenz), und dass er an allen anderen Stellen $c_{j \neq i}$ Einträge mit einem Wert von "0" aufweist.

Beispielsweise kann ein Vektor mit der Gestalt $(1,0,0)^T$ eine T1-gewichtete MRT-Sequenz, ein Vektor mit der Gestalt $(0,1,0)^T$ eine T2-gewichtete MRT-Sequenz, und ein Vektor mit der Gestalt $(0,0,1)^T$ eine PD-gewichtete MRT-Sequenz indizieren. Eine Misch-Gewichtung von z.B. 50% PD-gewichtet und 50% Tl-gewichtet kann somit durch einen Bedingungsvektor mit der Gestalt $(0.5, 0, 0.5)^T$ indiziert werden.

Der Bedingungsvektor kann auch eine oder mehrere weitere Bedingungen enthalten, beispielsweise eine Bedingung, welche ein zu untersuchendes Körperteil, einen Körperfettanteil, eine gewünschte Artefaktunterdrückung oder dergleichen indiziert.

Wie im Voranstehenden bereits erläutert wurde, können auch weitere Bedingungen für das bedingbare erzeugende künstliche neuronale Netzwerk 22 gesetzt werden, beispielsweise eine magnetische Feldstärke der durchzuführenden MRT-Sequenz. Es ist nämlich bekannt, dass sich beispielsweise die Repetitionszeit, TR, und die Echozeit, TE, ändern, wenn statt einer magnetischen Feldstärke von eineinhalb Tesla (1,5T) eine magnetische Feldstärke von drei Tesla (3T) eingestellt wird.

Das System 100 umfasst eine Ausgabeschnittstelle 30, über welche die erzeugten Einstellungsparameter 70 ausgebbar sind, beispielsweise an eine Anzeigevorrichtung 40 (z.B. einen Bildschirm oder Touchscreen) zum Anzeigen der erzeugten Einstellungsparameter 70 und/oder an eine MRT-Vorrichtung 50. Die Anzeigevorrichtung 40 und/oder die MRT-Vorrichtung 50 können auch von dem System 100 umfasst sein. Die MRT-Vorrichtung 50 kann derart ausgebildet sein, dass sie durch die von der Ausgabeschnittstelle 30 ausgegebenen Einstellungsparameter 70 automatisch eingestellt wird.

Dementsprechend stellt die vorliegende Erfindung auch ein Verfahren zum Anzeigen von optimalen Einstellungsparametern 70 sowie ein Verfahren zum automatischen Einstellen einer MRT-Vorrichtung 50 mit optimalen Einstellungsparametern 70 bereit.

Fig. 2 zeigt eine schematische Darstellung eines möglichen Aufbaus des trainierten bedingbaren erzeugenden künstlichen neuronalen Netzwerks 22, welches durch die Recheneinrichtung 20 des Systems 100 implementiert ist.

Entsprechend der im vorliegenden Beispiel verwendeten zwei Eingabedaten PD% und T1% ist eine Eingabeschicht 21 des bedingbaren erzeugenden künstlichen neuronalen Netzwerks 22 mit fünf Knoten vorgesehen: zwei Knoten (erste zwei Knoten von oben in Fig. 2) der Eingabeschicht 21 sind Eingabeknoten für PD% und T1% und drei Knoten (letzte drei Knoten von oben in Fig. 2) der Eingabeschicht 21 sind Eingabeknoten für die Einträge $c_i$ des Bedingungsvektors c.

Wie im Voranstehenden beschrieben, kann dabei der erste Eintrag c1 einen Prozentsatz einer T1-Gewichtung, der zweite Eintrag c2 einen Prozentsatz einer T2-Gewichtung und der dritte Eintrag c3 einen Prozentsatz einer PD-Gewichtung indizieren, und somit das bedingbare erzeugende künstliche neuronale Netzwerk 22 entsprechend bedingen, d.h. beeinflussen, um Einstellungsparameter 70 entsprechend der durch den Bedingungsvektor $c$ indizierten Prozentsätze zu erzeugen.

Für den Fall, dass dem bedingbaren erzeugenden künstlichen neuronalen Netzwerk 22 weitere Bedingungen als die Gewichtungs-Prozentsätze vorgegeben werden sollen, kann der Bedingungsvektor $c$ entsprechend weitere Einträge erhalten, wobei die Eingabeschicht 21 für jeden weiteren Eintrag des Bedingungsvektors $c$ mit einem weiteren Eingabeknoten ausgestattet ist. Beispielsweise könnte eine "1" in einem vierten Eintrag des Bedingungsvektors c bedeuten, dass eine MRT-Sequenz an einem menschlichen Kopf durchgeführt werden soll, eine "1" in einem fünften Eintrag des Bedingungsvektors c könnte bedeuten, dass eine MRT-Sequenz an einem menschlichen Herz durchgeführt werden soll, eine "1" in einem sechsten Eintrag des Bedingungsvektors c könnte bedeuten, dass eine MRT-Sequenz an einem unspezifizierten Körperteil vorgenommen werden soll, eine Wert in einem siebten Eintrag des Bedingungsvektors $c$ könnte einen Körperfettanteil eines Körpers, an dem die MRT-Sequenz durchzuführen ist, indizieren, und/oder dergleichen mehr.

Die Knoten der Eingabeschicht 21 sind über trainierbare Gewichte $W_h$ (engl. "weights") mit Knoten einer verborgenen Schicht 23 des bedingbaren erzeugenden künstlichen neuronalen Netzwerks 22 voll verbunden, d.h. die verborgene Schicht 23 ist eine vollverbundene Schicht (engl. "fully-connected layer"). Die Knoten der verborgenen Schicht 23 umfassen eine jeweilige Bias (engl. "bias") $b_h$ als trainierbaren Parameter sowie eine Aktivierungseinheit, z.B. eine Sigmoid-Einheit, wie in Fig. 2 angedeutet, eine tanh-Aktivierungseinheit oder dergleichen.

Vorteilhaft umfasst die verborgene Schicht 23 mehr als fünf Knoten, bevorzugt mehr als zehn Knoten, besonders bevorzugt mehr als zwanzig Knoten, beispielsweise vierzig Knoten oder mehr, z.B. achtundvierzig Knoten. Die Zahl von achtundvierzig hat sich in Versuchen als eine gute Balance zwischen Genauigkeit und Rechenaufwand herausgestellt.

Die verborgene Schicht 23 ist vorteilhaft voll mit einer Ausgabeschicht 25 verbunden, welche somit ebenfalls als vollverbundene Schicht ausgebildet ist. Die Ausgabeschicht 25 weist für die Verbindungen zwischen den Knoten der verborgene Schicht 23 und den Knoten der Ausgabeschicht 25 Gewichte $W_s$ als trainierbare Parameter auf.

Die Ausgabeschicht 25 besteht vorteilhaft aus je einem Knoten pro zu erzeugendem Einstellungsparameter 70. Im Fall des Erzeugens bloß der Echozeit, TE, und der Repetitionszeit, TR, besteht die Ausgabeschicht 25 somit vorteilhaft aus genau zwei Knoten. Die Knoten weisen jeweils eine Bias $b_s$ als trainierbaren Parameter sowie eine Aktivierungsfunktion auf, beispielsweise eine lineare Aktivierungsfunktion, wie in Fig. 2 angedeutet.

Es versteht sich, dass statt der genannten Eingabedaten 80 auch andere Eingabedaten verwendet werden können, beispielsweise ein vollständiges MRT-Protokoll oder eine MRT-Sequenz, zusammen mit einer Bedingung, z.B. einer magnetischen Feldstärke, einem zu untersuchenden Körperteil oder einer der anderen vorgenannten Bedingungen. Das bedingbare erzeugende künstliche neuronale Netzwerk 22 kann dazu ausgebildet sein, aus diesen Eingabedaten 80 entsprechend bedingte Einstellungsparameter 70 zu erzeugen, beispielsweise ein auf die in der Bedingung angegebene magnetische Feldstärke angepasstes MRT-Protokoll bzw. MRT-Sequenz.

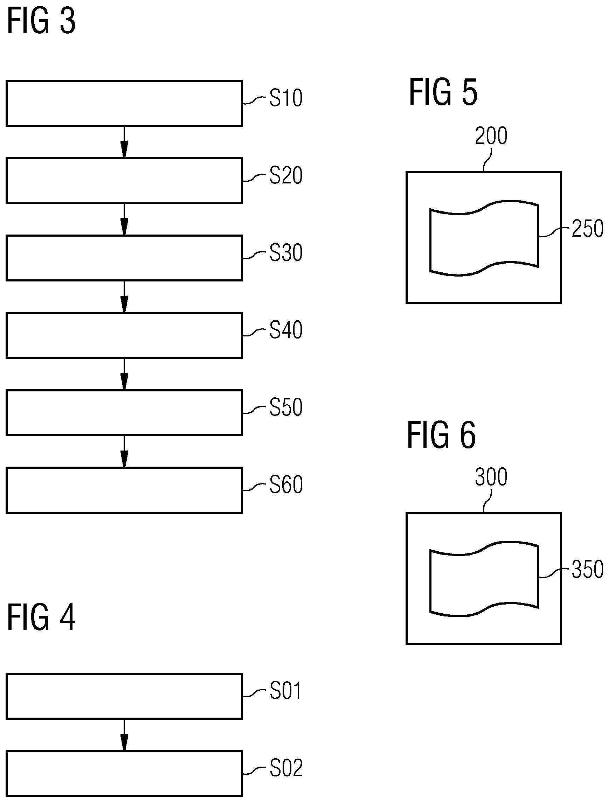

Fig. 3 zeigt ein schematisches Flussdiagramm zum Erläutern eines Verfahrens zum Herstellen eines Systems zum Erzeugen von Einstellungsparametern für eine durchzuführende MRT-Sequenz gemäß einer Ausführungsform des zweiten Aspekts der vorliegenden Erfindung, insbesondere zum Herstellen eines Systems gemäß dem ersten Aspekt der vorliegenden Erfindung. Das Verfahren gemäß Fig. 3 eignet sich insbesondere zum Herstellen des Systems 100 gemäß Fig. 1 und ist daher gemäß allen mit Bezug auf das System 100 beschriebenen Ausführungsformen, Optionen, Varianten und Weiterbildungen anpassbar und umgekehrt. Das Verfahren gemäß Fig. 3 wird teilweise unter Verwendung von Bezugszeichen der Fig. 1 und Fig. 2 beschrieben, ohne zwangsläufig auf das System 100 aus Fig. 1 und Fig. 2 eingeschränkt zu sein.

In einem Schritt S10 werden Trainingsdaten bereitgestellt, welche Eingabedaten 80 bezüglich der durchzuführenden MRT-Sequenz umfassen und welche entsprechende Einstellungsparameter 70 als Labels umfassen.

In einem Schritt S20 werden ein bedingbares erzeugendes künstliches neuronales Netzwerk 22 und ein bedingbares unterscheidendes künstliches neuronales Netzwerk bereitgestellt.

Das bedingbare erzeugende künstliche neuronale Netzwerk 22 kann insbesondere so beschaffen sein wie im Voranstehenden und im Nachfolgenden detailliert beschrieben.

Das bedingbare unterscheidende künstliche neuronale Netzwerk umfasst vorteilhaft eine Eingabeschicht mit ebenso vielen Knoten wie es der Summe der erzeugten Einstellungsparameter 70 und der Einträge des Bedingungsvektors c entspricht, im Beispiel von Fig. 2 also beispielsweise fünf Knoten.

Weiterhin umfasst das bedingbare unterscheidende künstliche neuronale Netzwerk eine verborgene Schicht mit beispielsweise zwischen dreißig und sechzig, bevorzug zwischen vierzig und fünfzig, besonders bevorzugt achtundvierzig Knoten. Eine Ausgabeschicht des bedingbaren unterscheidenden künstlichen neuronalen Netzwerks umfasst nur einen einzelnen Ausgabeknoten, dessen Ausgabe indiziert, ob das bedingbare unterscheidende künstliche neuronale Netzwerk einen Eingabevektor als "echt" (d.h. als zu den Trainingsdaten gehörig) oder als "falsch" (d.h. als von dem bedingbaren erzeugenden künstlichen neuronalen Netzwerk 22 erzeugt) klassifiziert, beispielsweise durch "0" für "echt" und "1" für "falsch".

In einem Schritt S30 wird das bedingbare erzeugende künstliche neuronale Netzwerk 22 unter Verwendung der bereitgestellten Trainingsdaten trainiert, um die Einstellungsparameter 70 für durchzuführende MRT-Sequenzen basierend auf den Eingabedaten 80 der Trainingsdaten zu erzeugen. Das bedingbare erzeugende künstliche neuronale Netzwerk ist dabei bedingbar, um Einstellungsparameter für zumindest jeweils eine T1-gewichtete MRT-Sequenz, eine T2-gewichtete MRT-Sequenz und eine Protondichte-gewichtete MRT-Sequenz zu erzeugen, und wird bei dem Trainieren S30 vorteilhaft auch, beispielsweise gleichmäßig, durch jede der möglichen Bedingungen bedingt, d.h. jeder der möglichen Bedingungen zu möglichst gleichen Anteilen unterworfen.

In einem Schritt S40 wird das bedingbare unterscheidende künstliche neuronale Netzwerk unter Verwendung der bereitgestellten Trainingsdaten und der von dem bedingbaren erzeugenden künstlichen neuronalen Netzwerk 22 erzeugten Einstellungsparameter 70 trainiert, wobei das bedingbare unterscheidende künstliche neuronale Netzwerk trainiert wird, um für jeden einzelnen Parametersatz zu unterscheiden, ob dieser zu den Trainingsdaten oder zu den von dem bedingbaren erzeugenden künstlichen neuronalen Netzwerk erzeugten Einstellungsparametern gehört.

Das Trainieren S40 umfasst, das bedingbare erzeugende künstliche neuronale Netzwerk 22 derart zu trainieren, die Einstellungsparameter 70 derart zu erzeugen, dass das bedingbare unterscheidende künstliche neuronale Netzwerk die Einstellungsparameter der Trainingsdaten nicht von denjenigen Einstellungsparameter 70 unterscheiden kann, welche das bedingbare erzeugende künstliche neuronale Netzwerk 22 erzeugt.

Mit anderen Worten wird das bedingbare erzeugende künstliche neuronale Netzwerk 22 als generativer Teil einer bedingbaren erzeugenden antagonistischen neuronalen Netzwerkstruktur (engl. "conditional generative adversarial neural network") trainiert, wobei z.B. die aus Mirza et al. oder Mao et al. oder auch aus anderen Quellen bekannten Varianten von (bedingbaren) erzeugenden antagonistischen neuronalen Netzwerkstrukturen angewendet werden können.

In einem Schritt S50 wird eine Eingabeschnittstelle 10 zum Empfangen von Eingabedaten 80 bezüglich durchzuführender MRT-Sequenzen bereitgestellt und eingerichtet.

In einem Schritt S60 wird eine Recheneinrichtung 20 zum Implementieren des trainierten bedingbaren erzeugenden künstlichen neuronalen Netzwerks 22 zum Erzeugen der Einstellungsparameter 70 für die durchzuführenden MRT-Sequenz basierend auf den empfangenen Eingabedaten 80 bereitgestellt und eingerichtet.

Fig. 4 zeigt ein schematisches Flussdiagramm, welches ein Verfahren zum Erzeugen von Einstellungsparametern für eine durchzuführende MRT-Sequenz gemäß einem dritten Aspekt der vorliegenden Erfindung illustriert. Das Verfahren gemäß Fig. 4 ist insbesondere mit dem System gemäß dem ersten Aspekt der vorliegenden Erfindung durchführbar und ist daher gemäß allen in Bezug auf das System gemäß dem ersten Aspekt (insbesondere in Bezug auf das System 100 aus Fig. 1) beschriebenen Modifikationen, Varianten, Optionen und Weiterbildungen anpassbar und umgekehrt.

In einem Schritt S01 werden Eingabedaten 80 bezüglich einer durchzuführenden MRT-Sequenz empfangen, beispielsweise wie in Bezug auf die Eingabeschnittstelle 10 und/oder die Eingabedaten 80 im Voranstehenden erläutert.

In einem Schritt S02 werden, unter Verwendung eines trainierten bedingbaren erzeugenden künstlichen neuronalen Netzwerks 22, Einstellungsparameter 70 für die durchzuführende MRT-Sequenz basierend auf den empfangenen Eingabedaten 80 erzeugt, insbesondere wie im Voranstehenden mit Bezug auf das bedingbare erzeugende künstliche neuronale Netzwerk 22 beschrieben wurde. Das bedingbare erzeugende künstliche neuronale Netzwerk 22 ist bedingbar, um Einstellungsparameter 70 für zumindest jeweils eine T1-gewichtete MRT-Sequenz, eine T2-gewichtete MRT-Sequenz und eine Protondichte-gewichtete MRT-Sequenz zu erzeugen.

Im Folgenden werden beispielhaft Programmcodeabschnitte angegeben, welche mögliche Varianten des bedingbaren erzeugenden künstlichen neuronalen Netzwerks 22 und/oder der bedingbaren erzeugenden antagonistischen Netzwerkstruktur erläutern sowie die Effektivität der vorliegenden Erfindung demonstrieren.

Die Programmcodeabschnitte sind in Google® TensorFlow® Version 1.4.0 verfasst, wobei das bedingbare erzeugende künstliche neuronale Netzwerk 22 selbstverständlich auch in anderen Programmiersprachen realisiert sein kann.

Der Übersichtlichkeit halber sind die Codeabschnitte in Input-Blöcke unterteilt, welche mit "In [x]" eingeleitet werden, wobei "x" für die Nummer des jeweiligen Input-Blocks steht. Wo zum schnelleren Verständnis vorteilhaft, wurden Kommentare eingefügt, der Code ist jedoch prinzipiell selbsterklärend.

```
In [1]:
import numpy as np
import matplotlib.pyplot as plt
%matplotlib inline


In [2]:
import tensorflow as tf
print ("TensorFlow r", tf.__version__)
TensorFlow r 1.4.0
#Dies gibt die verwendete Version von TensorFlow an
```

Vorteilhafte Parameterwertemengen, insbesondere für Einstellungsparameter 70, sowie Trainingsdaten zum Demonstrieren der Effizienz der vorliegenden Erfindung können etwa in der folgenden Weise definiert und erzeugt werden:

```
In [3]:
def ParameterWeightingIdx (TR, TE, T1=1084, T2=69):
        # Gewichtungsanteile N, T1, T2, N:
        SN = 1.0
        ST1 = np.abs (1.0 / (1.0—np.exp (TR/T1)))
        ST2 = np.abs (TE/T2)


        # Gewichtungsindizes (Gesamtsumme := 1.0)
        NWI = SN / (ST + ST2 + 1.0) # PD-Gewichtung
        T1WI = ST1 * NWI # T1-Gewichtung
        T2WI = ST2 * NWI # T2-Gewichtung


    return np.vstack ([T1WI, T2WI, NWI]).T
```

Hierbei stellen die "Gewichtungsindizes" NWI, T1WI und T2WI jeweils die als Dezimalbruch dargestellten Prozentsätze PD%, T1% und T2% dar, d.h.:
NWI=PD%/100, T1WI=T1%/100, und T2WI=T2%/100.

```
In [4]:
N = 600


# T1w
X1 = np.dot (np.random.rayleigh (1.0, [N, 2]), [[120., 8.]])
+ [600., 10.]
C1 = np.tile (np.eye (3) [0], N). reshape ((N, 3)) # [1. 0.
0.]
#C1 = ParameterWeightingIdx(X1[:, 1])


# T2w
X2 = np.dot (np.random.rayleigh (2.0, [N, 2]), [[-100., 0.],
[0., -6.]]) + [3000., 120.]
C2 = np.tile (np.eye (3)[1], N). reshape ((N,3)) # [0. 1. 0.]
#C2 = ParameterWeightingIdx(X2[;, 1])


# PDw
X3 = np.dot (np.random.rayleigh (2.0, [N, 2]), [[-100., 0.],
[-30., 6.]]) + [3000., 10.]
C3 = np.tile (np.eye (3) [2], N). reshape ((N,3)) # [0. 0. 1.]


X = np.concatenate ([X1, X2, X3]) # Value
C = np.concatenate ([C1, C2, C3]) # Label


In [5]:
train_data = np.concatenate ([X, C], 1)
T1mask = (np.logical_and(train_data[::,2] > train_data[::,3],
train_data[::, 2] > train
_data[::,4]))
T2mask = (np.logical_and(train_data[::,3] > train_data[::,2],
train_data[::3,] > train
_data[::,4]))
PDmask = (np.logical_and(train_data[::,4] > train_data[::,2],
train_data[::,4] > train
```

```
_data[::,3]))

        print (T1mask)

        [ True True True …, False False False]
```

In der Praxis werden die Trainingsdaten selbstverständlich nicht mit np.random.rayleigh zufallserzeugt, sondern aus Datensätzen mit bekannten Einstellungsparametern 70 entnommen.

Für das Erzeugen der bedingbaren erzeugenden antagonistischen Netzwerkstruktur kann beispielsweise die folgende Codestruktur verwendet werden:

```
In [8]:
def sampleZ(Ncol):
        return np.random.uniform(-1.0, 1.0, size=(Ncol,2))
#np.random.randn(Ncol,2)


def netG(z, c, reuse=False):
        with tf.variable_scope('generator', reuse=reuse):
            z_conditioned = tf.concat([z, c], 1)


        G_input = tf.contrib.layers.fully_connected(z_conditione
d, 48, activation_fn=tf.nn.tanh)
            G_output =
tf.contrib.layers.fully_connected(G_input, 2, activa-
tion_fn=None)
            return G_output


def netD(x,c, reuse=False):
        with tf.variable_scope('discriminator', reuse=reuse):
            x_conditioned = tf.concat([x, c], 1)
```

```
        D_input =
tf.contrib.layers.fully_connected(x_conditioned, 48, activa-
tion_fn=tf.nn.tanh)
        D_output =
tf.contrib.layers.fully_connected(D_input, 1, activa-
tion_fn=tf.nn.tanh)
        return D_output


In [9]:
z = tf.placeholder(tf.float32, shape=[None, 2],
name='G_input')
x = tf.placeholder(tf.float32, shape=[None, 2],
name='D_input')
c = tf.placeholder(tf.float32, shape=[None, 3],
name='C_label')


G_sample = netG(z, c)


D_real = netD(x, c)
D_fake = netD(G_sample, c, reuse=True)


D_var = tf.trainable_variables(scope='discriminator')
G_var = tf.trainable_variables(scope='generator')
```

Weiterhin werden eine Verlustfunktion (engl. "loss function", aber auch "cost function") D_loss für das bedingbare unterscheidende künstliche neuronale Netzwerk sowie eine Verlustfunktion G_loss für das bedingbare erzeugende künstliche neuronale Netzwerk 22 definiert und bei dem Trainieren S30, S40 der bedingbaren erzeugenden antagonistischen Netzwerkstruktur (d.h. insbesondere dessen erzeugenden Teils und dessen unterscheidenden Teils) im Rahmen der backpropagation verwendet.

Solche Verlustfunktionen D_loss und G_loss werden bevorzugt als Verlustfunktionen nach der Methode der kleinsten Quadrate ausgebildet (siehe auch Mao et al.), beispielsweise wie in dem folgenden Codeabschnitt ausgeführt:

```
In [10]:
# erzeugende antagonistische Netzwerkstruktur mit Verlust-
funktion der kleinsten Quadrate („Least-Squares GAN")
D_loss = 0.5*tf.reduce_mean(tf.square(D_real +
0.5*tf.reduce_mean(tf.square(D_fake))

G_loss = 0.5*tf.reduce_mean(tf.square(D_fake -1))

G_solver = tf.train.AdamOptimizer(learning_rate=1.e-
5).minimize(G_loss, var_list=G_va
r, name='G_solver')

D_solver = tf.train.AdamOptimizer(learning_rate=5.e-
5).minimize(D_loss, var_list=D_va
r, name='D_solver')
```

Mit "Tensorboard" kann eine graphische Darstellung erzeugt und bearbeitet werden, etwa wie in dem nachfolgenden Codeabschnitt ausgeführt:

```
In [11]:
tf.summary.histogram('D_real', D_real)
tf.summary.histogram(D_'fake', D_fake)

tf.summary.scalar('D_loss', D_loss)
tf.summary.scalar('G_loss', G_loss)

[tf.summary.histogram(ii.name.replace(':', '_'), ii) for ii
in G_var]
```

```
[tf.summary.histogram(jj.name.replace(':', '_'), jj) for jj
in D_var]


Out [11]:
[<tf.Tensor 'discriminator/fully_connected/weights_0:0'
shape=() dtype=string>,
tf.Tensor 'discriminator/fully_connected/biases_0:0' shape=()
dtype=string>,
tf.Tensor 'discriminator/fully_connected_1/weights_0:0'
shape=() dtype=string>,
tf.Tensor 'discriminator/fully_connected_1/biases_0:0'
shape=() dtype=string>]
```

Das Trainieren S30, S40 der erzeugenden antagonistischen Netzwerkstruktur erfolgt beispielsweise wie in dem nachfolgenden Codeabschnitt ausgeführt:

```
In [12]:
PLOT_INLINE = True


In [13]:
training_size = train_data.shape[0]
batch_size = 100


In [14]:
sess = tf.Session()


# TensorBoard – tensorboard --logdir=
summary_collection = tf.summary.merge_all()
tf_writer =
tf.summary.FileWriter(".\\tmp\\Image_Weighting_CGAN",
sess.graph)

    # Initialisieren
    sess.run(tf.global_variables_initializer())
```

Anschließend können die Ergebnisse etwa gemäß dem nachfolgenden Codeabschnitt angezeigt werden:

```
In[15]:
def plot_generated_results(batch_size, label=True, fig-
size=(10,5)):
    fig, ax = plt.subplots(1,1, figsize=figsize)

    X = np.dot(train_data[:,:2]+.5, [[3000., 0.], [0.,
120.]])

    ax.scatter(X[:, 0],X[:,1], s=0.1)

C1 = np.tile(np.array([1.0, 0.0,
0.0]),batch_size).reshape(batch_size,3)
C2 = np.tile(np.array([0.0, 1.0,
0.0]),batch_size).reshape(batch_size,3)
C3 = np.tile(np.array([0.0, 0.0,
1.0]),batch_size).reshape(batch_size,3)

    T1W = np.asarray(sess.run([G_sample], feed_dict={z: sam-
pleZ(batch_size), c: C1}))
    T1W = np.squeeze(T1W)
    T1W = np.dot(T1W+.5, [[3000., 0.],[0., 120.]])

    ax.scatter(T1W[:,0],T1W[:,1], s=1.2, c='orange')

    T2W = np.asarray(sess.run([G_sample], feed_dict={z: sam-
pleZ(batch_size), c: C2}))
    T2W = np.squeeze(T2W)
    T2W = np.dot(T2W+.5, [[3000., 0.],[0., 120.]])
```

```
ax.scatter(T2W[:,0],T2W[:,1], s=1.2, c='green')


    PDW = np.asarray(sess.run([G_sample], feed_dict={z: sam-
pleZ(batch_size), c: C3}))
    PDW = np.squeeze(PDW)
    PDW = np.dot(PDW+.5, [[3000., 0.],[0., 120.]])


    ax.scatter(PDW[:,0],PDW[:,1], s=1.2, c='red')


    if label:
        fig.suptitle('Generated Data (T1W, T2W, PDW)',
fontweight='bold')


        ax.set_xlabel('TR [ms]', fontweight='bold')
        ax.set_ylabel('TE [ms]', fontweight='bold')
        ax.legend(['REF', 'T1W', 'T2W', 'PDW'], mark-
erscale=5., loc='upper left')


    else:
        ax.set_xticks([])
        ax.set_yticks([])
    plt.show()
```

Es versteht sich, dass die aufgeführten Codeabschnitte in vorteilhaften Ausführungsformen verwendet werden können, wobei eine Vielzahl von alternativen Realisierungsmöglichkeiten besteht.

Fig. 5 zeigt eine schematische Darstellung eines Computerprogrammprodukts 200 gemäß einer Ausführungsform des vierten Aspekts der vorliegenden Erfindung. Das Computerprogrammprodukt 200 umfasst ausführbaren Programmcode 250, welcher dazu ausgebildet ist, das Verfahren gemäß einer Ausführungsform des dritten Aspekts der vorliegenden Erfindung durchzuführen, wenn er ausgeführt wird.

Fig. 6 zeigt eine schematische Darstellung eines nichtflüchtigen Datenspeichermediums 300 gemäß einer Ausführungsform des fünften Aspekts der vorliegenden Erfindung. Das Datenspeichermedium 300 umfasst ausführbaren Programmcode 350, welcher dazu ausgebildet ist, das Verfahren gemäß einer Ausführungsform des dritten Aspekts der vorliegenden Erfindung durchzuführen, wenn er ausgeführt wird.

In der vorangegangenen detaillierten Beschreibung sind verschiedene Merkmale zur Verbesserung der Stringenz der Darstellung in einem oder mehreren Beispielen zusammengefasst worden. Es sollte dabei jedoch klar sein, dass die obige Beschreibung lediglich illustrativer, keinesfalls jedoch beschränkender Natur ist. Sie dient der Abdeckung aller Alternativen, Modifikationen und Äquivalente der verschiedenen Merkmale und Ausführungsbeispiele. Viele andere Beispiele werden dem Fachmann aufgrund seiner fachlichen Kenntnisse in Anbetracht der obigen Beschreibung sofort und unmittelbar klar sein.

Die Ausführungsbeispiele wurden ausgewählt und beschrieben, um die der Erfindung zugrundeliegenden Prinzipien und ihre Anwendungsmöglichkeiten in der Praxis bestmöglich darstellen zu können. Dadurch können Fachleute die Erfindung und ihre verschiedenen Ausführungsbeispiele in Bezug auf den beabsichtigten Einsatzzweck optimal modifizieren und nutzen.

**Patentansprüche**

1. System (100) zum Erzeugen von Einstellungsparametern (70) für eine durchzuführende Magnetresonanztomographie-(MRT-)Sequenz, umfassend:

   eine Eingabeschnittstelle (10), welche dazu eingerichtet ist, Eingabedaten (80) bezüglich der durchzuführenden MRT-Sequenz zu erhalten;
   einer Recheneinrichtung (20), welche dazu ausgebildet ist, ein bedingbares erzeugendes künstliches neuronales Netzwerk (22), zu implementieren,
   wobei das bedingbare erzeugende künstliche neuronale Netzwerk (22) dazu ausgebildet und trainiert ist, basierend auf den von der Eingabeschnittstelle (10) empfangenen Eingabedaten (80) Einstellungsparameter (70) für die durchzuführende MRT-Sequenz zu erzeugen;
   wobei das bedingbare erzeugende künstliche neuronale Netzwerk (22) bedingbar ist, um Einstellungsparameter (70) für zumindest jeweils eine T1-gewichtete MRT-Sequenz, eine T2-gewichtete MRT-Sequenz und eine Protondichte-(PD-)gewichtete MRT-Sequenz zu erzeugen.

2. System (100) nach Anspruch 1,
   wobei die Eingabedaten (80) zumindest einen der folgenden Prozentsätze umfassen oder indizieren:

   - einen Prozentsatz (T1%), zu welchem die durchzuführende MRT-Sequenz Tl-gewichtet sein soll
   - einen Prozentsatz, zu welchem die durchzuführende MRT-Sequenz T2-gewichtet sein soll
   - einen Prozentsatz (PD%), zu welchem die durchzuführende MRT-Sequenz Protondichte-gewichtet sein soll.

3. System (100) nach Anspruch 1 oder 2,
   wobei die Einstellungsparameter (70) eine Repetitionszeit (TR) und eine Echozeit (TE) der durchzuführenden MRT-Sequenz umfassen.

4. System (100) nach einem der Ansprüche 1 bis 3,
   wobei die Einstellungsparameter (70) einen Turbofaktor und/oder eine Bandbreite der durchzuführenden MRT-Sequenz umfassen.

5. System (100) nach einem der Ansprüche 1 bis 4,
   wobei das bedingbare erzeugende künstliche neuronale Netzwerk (22) zusätzlich bedingbar ist durch mindestens eine der folgenden Bedingungen:

   - eine magnetische Feldstärke der durchzuführenden MRT-Sequenz,
   - eine Information über einen Ort und/oder eine Ausrichtung einer Schnittebene durch einen Körper, an welchem die MRT-Sequenz durchzuführen ist,
   - eine Information über einen Ort und/oder einen Körperteil eines Körper, an welchem die MRT-Sequenz durchzuführen ist,
   - einen Körperfettanteil,
   - eine Artefaktunterdrückung.

6. System (100) nach einem der Ansprüche 1 bis 5,
   wobei das bedingbare erzeugende künstliche neuronale Netzwerk (22) als Teil einer bedingbaren erzeugenden antagonistischen Netzwerkstruktur trainiert wurde, wobei die bedingbare erzeugende antagonistische Netzwerkstruktur das bedingbare erzeugende künstliche neuronale Netzwerk (22) als ihren erzeugenden Teil umfasst und ein bedingbares unterscheidendes künstliches neuronales Netzwerk als ihren unterscheidenden Teil umfasst.

7. System (100) nach einem der Ansprüche 1 bis 6,
   wobei das bedingbare erzeugende künstliche neuronale Netzwerk (22) aus einer Eingabeschicht (21), einer Ausgabeschicht (25) und einer dazwischenliegenden verborgenen Schicht (23) besteht.

8. System (100) nach Anspruch 7,
   wobei das bedingbare erzeugende künstliche neuronale Netzwerk (22) eine Eingabeschicht (21), eine Ausgabeschicht (25) und mindestens eine verborgene Schicht (23) umfasst, und wobei die Eingabeschicht die mindestens eine verborgene Schicht zumindest einen Teil eines Auto-Encoder-Netzwerk bilden.

9. System (100) nach einem der Ansprüche 1 bis 8,
   wobei das bedingbare erzeugende künstliche neuronale Netzwerk (22) als Multischicht-Perzeptron ausgebildet ist.

10. Verfahren zum Herstellen eines System (100) zum Erzeugen von Einstellungsparametern (70) für eine durchzuführende MRT-Sequenz, umfassend:

   Bereitstellen (S10) von Trainingsdaten, welche Eingabedaten (80) bezüglich der durchzuführenden MRT-Sequenz umfassen und welche entsprechende Einstellungsparameter (70) als Labels umfassen;
   Bereitstellen (S20) eines bedingbaren erzeugenden künstlichen neuronalen Netzwerks (22) und eines bedingbaren unterscheidenden künstlichen neuronalen Netzwerks;
   Trainieren (S30) des bedingbaren erzeugenden künstlichen neuronalen Netzwerks (22) unter Verwendung der bereitgestellten Trainingsdaten, wobei das bedingbare erzeugende künstliche neuronale Netzwerk (22) trainiert wird, die Einstellungsparameter (70) für durchzuführende MRT-Sequenzen basierend auf den Eingabedaten (80) der Trainingsdaten zu erzeugen;
   wobei das bedingbare erzeugende künstliche neuronale Netzwerk (22) bedingbar ist, um Einstellungsparameter (70) für zumindest jeweils eine T1-gewichtete MRT-Sequenz, eine T2-gewichtete MRT-Sequenz und eine Protondichte-gewichtete MRT-Sequenz zu erzeugen;
   Trainieren (S40) des bedingbaren unterscheidenden künstlichen neuronalen Netzwerk unter Verwendung der bereitgestellten Trainingsdaten und der von dem bedingbaren erzeugenden künstlichen neuronalen Netzwerk (22) erzeugten Einstellungsparameter (70), wobei das bedingbare unterscheidende künstliche neuronale Netzwerk trainiert wird, um für jeden einzelnen Parametersatz zu unterscheiden, ob dieser zu den Trainingsdaten oder zu den von dem bedingbaren erzeugenden künstlichen neuronalen Netzwerk (22) erzeugten Einstellungsparametern (70) gehört;
   und wobei das Trainieren (S30) des bedingbaren erzeugenden künstlichen neuronalen Netzwerks (22) umfasst, das bedingbare erzeugende künstliche neuronale Netzwerk (22) derart zu trainieren, die Einstellungsparameter (70) derart zu erzeugen, dass das bedingbare unterscheidende künstliche neuronale Netzwerk (22) die Trainingsdaten nicht von den von dem bedingbaren erzeugenden künstlichen neuronalen Netzwerk (22) erzeugten Einstellungsparametern (70) unterscheiden kann;
   Bereitstellen und Einrichten (S50) einer Eingabeschnittstelle (10) zum Empfangen von Eingabedaten (80) bezüglich durchzuführender MRT-Sequenzen; und
   Bereitstellen und Einrichten (S60) einer Recheneinrichtung (20) zum Implementieren des trainierten bedingbaren erzeugenden künstlichen neuronalen Netzwerks zum Erzeugen der Einstellungsparameter (70) für die durchzuführenden MRT-Sequenz basierend auf den empfangenen Eingabedaten (80).

11. Verfahren nach Anspruch 10,
   wobei das Trainieren (S30) des bedingbaren erzeugenden künstlichen neuronalen Netzwerks (22) und/oder das Trainieren (S40) des bedingbaren unterscheidenden künstlichen neuronalen Netzwerks unter Verwendung einer Verlustfunktion durchgeführt wird, welche auf der Methode der kleinsten Quadrate basiert.

12. Verfahren zum Erzeugen von Einstellungsparametern (70) für eine durchzuführende MRT-Sequenz, umfassend:

   Empfangen (S01) von Eingabedaten (80) bezüglich der durchzuführenden MRT-Sequenz;
   Erzeugen (S02), unter Verwendung eines trainierten bedingbaren erzeugenden künstlichen neuronalen Netzwerks (22), von Einstellungsparametern (70) für die durchzuführende MRT-Sequenz basierend auf den empfangenen Eingabedaten (80);
   wobei das bedingbare erzeugende künstliche neuronale Netzwerk (22) bedingbar ist, um Einstellungsparameter (70) für zumindest jeweils eine T1-gewichtete MRT-Sequenz, eine T2-gewichtete MRT-Sequenz und eine Protondichte-gewichtete MRT-Sequenz zu erzeugen.

13. Verfahren nach Anspruch 12,
   wobei die Einstellungsparameter (70) eine Repetitionszeit (TR), eine Echozeit (TE), einen Turbofaktor und/oder eine Bandbreite der durchzuführenden MRT-Sequenz umfassen.

14. Verfahren nach einem der Ansprüche 12 oder 13,
   wobei das bedingbare erzeugende künstliche neuronale Netzwerk (22) zusätzlich bedingt wird durch mindestens eine der folgenden Bedingungen:

- eine magnetische Feldstärke der durchzuführenden MRT-Sequenz,
- eine Information über einen Ort und/oder eine Ausrichtung einer Schnittebene durch einen Körper, an welchem die MRT-Sequenz durchzuführen ist,
- eine Information über einen Ort und/oder einen Körperteil eines Körper, an welchem die MRT-Sequenz durchzuführen ist,
- einen Körperfettanteil,
- eine Artefaktunterdrückung.

15. Nichtflüchtiges Datenspeichermedium (300), umfassend ausführbaren Programmcode (350), welcher derart ausgebildet ist, dass er, wenn er ausgeführt wird, das Verfahren gemäß einem der Ansprüche 11 bis 14 ausführt.

EP 3 598 161 A1

# FIG 1

# FIG 2

## FIG 3

S10

S20

S30

S40

S50

S60

## FIG 4

S01

S02

## FIG 5

200

250

## FIG 6

300

350

Europäisches
Patentamt

European
Patent Office

Office européen
des brevets

## EUROPÄISCHER RECHERCHENBERICHT

**Nummer der Anmeldung**

EP 18 18 4274

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 100 55 417 A1 (SIEMENS AG) 23. Mai 2002 (2002-05-23) | 1-15 | INV. G01R33/54 |
| Y | * Abbildung 2 * <br> * Absatz [0018] * <br> * Absatz [0025] * <br> * Ansprüche 1,4,5 * <br> ----- | 10,11 | G06N3/04 |
| X | EP 3 282 271 A1 (SIEMENS HEALTHCARE GMBH [DE]) 14. Februar 2018 (2018-02-14) <br> * Absatz [0005] * <br> * Absatz [0013] - Absatz [0023] * <br> * Absatz [0059] - Absatz [0061] * <br> ----- | 1-15 | |
| A | CHAPTER 15 4 ED - HAACKE E M: "Contrast Mechanisms in MRI and Contrast Maximization, Chapter 15: Signal, Contrast and Noise", 15. Juni 1999 (1999-06-15), MAGNETIC RESONANCE IMAGING - PHYSICAL PRINCIPLES AND SEQUENCE DESIGN, WILEY-LISS, NEW YORK, NY [U.A.], PAGE(S) 352 - 363, XP002718470, ISBN: 0-471-35128-8 <br> * Abschnitt "15.4 Contrast Mechanisms in MRI and Contrast Maximization" * <br> ----- | 1-15 | |
| A,D | IAN J GOODFELLOW ET AL: "Generative Adversarial Nets", PROCEEDINGS OF THE 2014 CONFERENCE ON ADVANCES IN NEURAL INFORMATION PROCESSING SYSTEMS, Bd. 27, 31. Dezember 2014 (2014-12-31), Seiten 2672-2680, XP055537898, <br> * Abschnitt "Abstract" * <br> * Abschnitt "3 Adversarial nets" * <br> * Abschnitt "6 Advantages and disadvantages" * <br> ----- | 1-15 | |

**RECHERCHIERTE SACHGEBIETE (IPC)**

G01R
G06N

-/--

Der vorliegende Recherchenbericht wurde für alle Patentansprüche erstellt

| Recherchenort | Abschlußdatum der Recherche | Prüfer |
|---|---|---|
| München | 18. Januar 2019 | Durst, Markus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

Seite 1 von 2

Europäisches
Patentamt

European
Patent Office

Office européen
des brevets

**EUROPÄISCHER RECHERCHENBERICHT**

Nummer der Anmeldung

EP 18 18 4274

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | Mehdi Mirza ET AL: "Conditional generative adversarial nets", arXiv:1411.1784v1 [cs.LG], 6. November 2014 (2014-11-06), XP055501175, Gefunden im Internet: URL:https://arxiv.org/abs/1411.1784v1 [gefunden am 2018-08-21] | 10,11 | |
| A | * Abschnitt "Abstract" * <br> * Abschnitt "3 Conditional Adversarial Nets" * <br> ----- | 1-9, 12-15 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 18. Januar 2019 | Durst, Markus |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes
    Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 18 18 4274

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-01-2019

| Im Recherchenbericht angeführtes Patentdokument | Datum der Veröffentlichung | Mitglied(er) der Patentfamilie | Datum der Veröffentlichung |
|---|---|---|---|
| DE 10055417 A1 | 23-05-2002 | CN 1353316 A<br>DE 10055417 A1<br>JP 2002191577 A<br>US 2002087066 A1 | 12-06-2002<br>23-05-2002<br>09-07-2002<br>04-07-2002 |
| EP 3282271 A1 | 14-02-2018 | CN 107693017 A<br>EP 3282271 A1<br>US 2018038930 A1 | 16-02-2018<br>14-02-2018<br>08-02-2018 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **R.W.BROWN et al.** Magnetic Resonance Imaging: Physical Principles and Sequence Design. Wiley-Blackwell, 23. Juni 2014
- Generative Adversarial Nets. **I. J. GOODFELLOW et al.** Advances in Neural Information Processing Systems. 2014, vol. 27
- **M. MIRZA et al.** Conditional Generative Adversarial Nets. *arXiv preprint arXiv:1411.1784v1,* 06. November 2014, https://arxiv.org/abs/1411.1784
- **X. MAO et al.** Least Squares Generative Adversarial Networks. *arXiv preprint arXiv:1611.04076v3,* 13. November 2016